# EUROPÄISCHE PATENTSCHRIFT

(11) **EP 3 718 517 B1**
(45) Veröffentlichungstag und Bekanntmachung des Hinweises auf die Patenterteilung: **22.03.2023**
(21) Anmeldenummer: 19020243.2
(22) Anmeldetag: 01.04.2019
(51) Int. Cl.: A61F 5/455, A47K 11/12, A61G 9/00

(54) **AUFFANGVORRICHTUNG FÜR AUSSCHEIDUNGEN**
COLLECTING DEVICE FOR OFFCUTS
DISPOSITIF DE COLLECTE POUR PRÉCIPITATIONS

(43) Veröffentlichungstag der Anmeldung: 07.10.2020
(73) Patentinhaber: Hohmuth, Horst, 89073 Ulm (DE)
(72) Erfinder: Hohmuth, Horst, 89073 Ulm (DE)
(74) Vertreter: Kienle, Thomas

(56) Entgegenhaltungen:
- US-A- 3 724 461
- US-A1- 2002 193 762
- US-A1- 2010 278 456
- US-A1- 2018 185 227

## Beschreibung

Die vorliegende Erfindung betrifft eine Auffangvorrichtung für Ausscheidungen.

### Stand der Technik

Plötzlicher und nicht kontrollierbarer Harndrang kann für Betroffene, wenn sie zum Beispiel im Stau stehen oder alle Toiletten belegt sind, zu einer persönlich entwürdigenden Situation führen. Ebenso kann plötzliches unkontrollierbares Erbrechen beschämend und unangenehm sein. Es gibt bereits einfach konstruierte, preiswerte Einweg-Vorrichtungen zum Auffangen von Harn oder Erbrochenem für Kliniken. Solche Vorrichtungen beinhalten meist ein Geliermittel, das aufgefangenen Urin geliert und dadurch ein Auslaufen verhindert.

Aus der US 2018 185227 ist etwa ein Einweg-Kunststoffbeutel zum Auffangen und Abmessen von Urin oder Erbrochenem für Krankenhäuser beschrieben. Der Einweg-Kunststoffbeutel hat einen auf die weibliche Anatomie angepassten flexiblen Stutzen und einen wiederöffenbaren Zip-Verschluss. Der Einweg-Kunststoffbeutel beinhaltet ein Geliermittel um das aufgefangene Material zu gelieren und um anschließend das Volumen des Materials abmessen zu können.

Aus der US 2016 088983 ist ein flacher Beutel bekannt, der einen auffaltbaren, festen Kunststoffkragen mit einem integrierten Schnabel aufweist. Der Schnabel ist an die weibliche Anatomie angepasst, um effektiv Urin in den Beutel zu leiten.

Aus der US 2012 222208 ist ein tragbarer Urinbeutel bekannt, der einen Kunststoff-Kragenteil und -Beutelteil umfasst, die einstückig oder miteinander thermoverschweißt sind. Wird der Kunststoffkragenteil an seinen distalen Kanten zwischen Zeigefinger und Daumen zusammengedrückt, so bildet er eine Einfüllöffnung. Wird der Kunststoffkragenteil losgelassen, verschließen mehrere ineinandergreifende Leisten den Urinbeutel wiederöffenbar. Der Kunststoffkragenteil weist zwei ebene Ränder auf. Der tragbare Urinbeutel beinhaltet ein Geliermittel, um aufgefangenen Urin zu gelieren bzw. aufzusaugen. Außerdem beinhaltet der tragbare Urinbeutel ein geruchsbeseitigendes Mittel.

Aus der US 2010 318044 ist ein flach faltbarer Urinbeutel bekannt. Der flach faltbare Urinbeutel besteht aus Synthetikharz und weist einen Dichtverschluss auf, der durch Verformen des Urinbeutels geöffnet oder verschlossen werden kann. Ein Trichter mit mehreren ausfaltbaren Laschen bzw. Flügel ist an einer Öffnung des Urinbeutels befestigt und so ausgelegt, dass die Öffnung je nach Faltung der Laschen bzw. Flügel geöffnet oder geschlossen ist. Der Urinbeutel beinhaltet ein wasseraufsaugendes Mittel, um das Auslaufen von Urin zu verhindern.

Aus der US 2010/278456 A1 ist ein trag- und verschließbarer Behälter zum Auffangen von menschlichen Abfallstoffen offenbart. Der Behälter nimmt die Abfallstoffe durch eine Öffnung auf und fängt sie in einer unteren Kammer auf. Der Behälter umfasst ein Ventil, das über gekreuzte Bänder mit der unteren Kammer verbunden ist. Füllt sich die untere Kammer, so verschließen die Bänder das Ventil. Zwischen der Öffnung und der Kammer kann ein abnehmbareres Vorspannelement vorgesehen sein, das die Öffnung öffenbar verschließt. An der Öffnung ist ein Klebestreifen mit abnehmbarer Abdeckung zum Verschließen der Öffnung angeordnet. Ferner kann der Behälter eine flexible Randleiste umfassen, die als Trichter für die Öffnung dient. Die Randleist weist einen eigenen Verschluss an ihrer Oberseite auf.

Aus US 3,724,461 ist ein Urinbeutel mit einem Hauptbehälter und zwei einander gegenüberliegenden Wänden bekannt. Die Wände sind seitlich heißversiegelt und bilden einen ovalen, langegestreckten, oben offenen Beutel. Der Urinbeutel umfasst einen Einlass- und Ventilbereich. Zwei sich gegeneinander wölbende und gegenüberliegende Federelemente erstrecken sich entlang von Wandungen. Die Federelemente können mit den Fingern so gedrückt werden, dass sie sich auseinander wölben. Die Wandungen schließen ferner einen nach innen gefalteten Randbereich ab.

Die US 2002/0193762 A1 offenbart eine handgehaltene, wegwerfbare, tragbare Urinalvorrichtung mit einem ersten verschließbaren äußeren Beutel, einem Trichter und einem flexiblen, erweiterten Abschnitt, der den Beutel mit dem Trichter verbindet. Der Beutel umfasst ferner einen Ziplock-Verschluss .

Somit besteht die Aufgabe der Erfindung darin, eine kompakt zusammenfaltbare und zugleich sicher und einfach öffen- und verschließbare Auffangvorrichtung für Ausscheidungen bereitzustellen.

### Offenbarung der Erfindung

Erfindungsgemäß wird eine Auffangvorrichtung für Ausscheidungen gemäß Anspruch 1 zur Verfügung gestellt. Bevorzugte Ausführungsformen werden in den abhängigen Ansprüchen definiert.

Ausscheidungen umfassen dabei insbesondere Urin und Erbrochenes. Der Auffangbeutel umfasst vorzugsweise ein intransparentes, fluiddichtes Kunststoffmaterial und kann ca. 500 ml fassen. Die Intransparenz ermöglicht es aufgefangene Ausscheidungen dezent und unauffällig im Auffangbeutel zu halten. Die Fluiddichtigkeit verhindert unangenehme Gerüche sowie ein Auslaufen, auch ohne die bekannten Geliermittel. Der Auffangbeutel ist vorzugsweise zylinderförmig und weist an einer Zylinderdeckelseite den ersten Auffangbeutel-Öffnungsbereich auf. Der Auffangbeutel hat insbesondere eine Längsachse. Der erste Auffangbeutel-Öffnungsbereich kann eine erste Auffangbeutel-Öffnung umgeben bzw. einen Rand derselben bilden.

Die flexible Manschette umfasst vorzugsweise eine Ummantelung bzw. ein röhrenförmiges oder zylindrisches Teil, das insbesondere entlang der Längsachse angeordnet ist. Die flexible Manschette kann aus einem stabileren bzw. steiferen Material als der Auffangbeutel gebildet sein. Dennoch ist es von Vorteil, wenn die flexible Manschette flexibel verformbar bzw. biegbar ist. Der erste Manschetten-Öffnungsbereich ist vorzugsweise an einer Zylinderdeckelseite der flexiblen Manschette ausgebildet, während der zweite Manschetten-Öffnungsbereich an einer anderen, d.h. gegenüberliegenden, Zylinderdeckelseite ausgebildet sein kann. Der erste und der zweite Manschetten-Öffnungsbereich umgeben vorzugsweise je eine Manschetten-Öffnung bzw. bilden Ränder derselben. Ein Durchmesser der flexiblen Manschette entspricht im Wesentlichen einem Durchmesser des Auffangbeutels. Der Auffangbeutel ist vorzugsweise mit der flexiblen Manschette verbunden, insbesondere fluiddicht verklebt. Die flexible Manschette und der Auffangbeutel können einen flachgedrückten Zustand einnehmen, in dem sie eine Rechteckform aufweisen. Die flexible Manschette hat in dem flachgedrückten Zustand vorzugsweise eine quadratische Form, noch bevorzugter eine Abmessung von 9 mal 9 cm. Die Manschette umfasst ein Kartonmaterial oder ein Kunststoffmaterial. Die Manschette dient insbesondere als Griff zum Festhalten der Auffangvorrichtung durch einen Benutzer, zu einem Einleiten bzw. Führen von Ausscheidungen in den Auffangbeutel und zu einem Stabilisieren des ersten Auffangbeutel-Öffnungsbereichs. Die Manschette kann die Außenabmessung der Auffangvorrichtung in einem zusammengefalteten Zustand definieren und die zusammengefaltete Auffangvorrichtung stabilisieren bzw. stützen. Die flexible Manschette kann ferner auf ihrer Außenseite bedruckt sein. Die flexible Manschette kann so vorteilhaft als Verpackungsmittel eingesetzt werden.

Die Federspange umfasst ein Federelement mit zwei Schenkeln, die in einem entspannten Zustand im Wesentlichen parallel zueinander angeordnet sind und in einem gestauchten Zustand eine O-Form bzw. eine Ellipsen-Form bilden. Die Federspange kann auf der Außenseite, d.h. Zylinderaußenwand, der flexiblen Manschette oder einer Innenseite, d.h. Zylinderinnenwand, der flexiblen Manschette angeordnet sein oder einstückig mit der flexiblen Manschette ausgebildet sein. Vorzugsweise ist die Federspange auf der Innenseite der flexiblen Manschette umlaufend am zweiten Manschetten-Öffnungsbereich der Manschette aufgeklebt. Entlang der Längsachse misst die Federspange beispielsweise ca. 2 cm. Im entspannten Zustand kann die Federspange die flexible Manschette und/oder den Einfülltrichter und/oder den Auffangbeutel flach formen bzw. ziehen oder drücken wodurch die erfindungsgemäße Auffangvorrichtung verschlossen werden kann. Der Zustand wird im Folgenden auch als flachgedrückter Zustand bezeichnet. Im gestauchten Zustand kann die Federspange die Manschette und/oder den Einfülltrichter und/oder den Auffangbeutel zylinderartig formen, wodurch die erfindungsgemäße Auffangvorrichtung geöffnet werden kann. Ein Benutzer kann durch Drücken der Federspange an ihren distalen Enden, etwa zwischen Daumen und Zeigefinger derselben Hand, den gestauchten Zustand und durch Loslassen den entspannten Zustand herbeiführen. So kann die flexible Manschette bzw. die erfindungsgemäße Auffangvorrichtung einhändig sicher und einfach geöffnet und wieder verschlossen werden, um zum Beispiel ein zwischenzeitliches Auslaufen von aufgefangenen Ausscheidungen zu verhindern und/oder unangenehme Gerüche von aufgefangenen Ausscheidungen zu verringern. Dieser Mechanismus wird im Folgenden auch als Automatikverschluss bezeichnet.

Der Einfülltrichter umfasst vorzugsweise ein Wachspapiermaterial, insbesondere ein nanobeschichtetes Wachspapiermaterial, um vorteilhaft wasser- bzw. schmutzabweisend zu sein. Der erste Einfülltrichter-Öffnungsbereich umgibt insbesondere eine erste Einfülltrichter-Öffnung bzw. bildet einen Rand derselben. Der Einfülltrichter ist vorzugsweise entlang der Längsachse angeordnet; er kann im gestauchten Zustand eine Zylinderform aufweisen und hat einen Durchmesser, der in etwa dem von flexibler Manschette und Auffangbeutel entspricht.

Die hier beschriebenen Zylinderformen umfassen ovale Zylinderformen. Einander gegenüberliegende Öffnungen meint im Wesentlichen deckungsgleiche Öffnungen, die in etwa parallel zueinander und entlang der gemeinsamen Längsachse angeordnet sind.

Die Auffangvorrichtung umfasst ein flexibles Zwischenelement, das den Einfülltrichter mit dem zweiten Manschetten-Öffnungsbereich verbindet. Das flexible Zwischenelement umfasst ein fluiddichtes, biegsames bzw. flexibles Kunststoffband, das an der Innenseite der flexiblen Manschette und an der Innenseite des Einfülltrichters angeordnet ist.

Das flexible Zwischenelement ist mit dem zweiten Manschetten-Öffnungsbereich ablösbar verbunden. Des Weiteren ist bevorzugt, dass die flexible Manschette ein Verschlussmittel umfasst, an dem das flexible Zwischenelement ablösbar verbunden sein kann. Wird das flexible Zwischenelement abgelöst, kann es das Verschlussmittel freilegen. Das Verschlussmittel kann mit sich selbst in Kontakt gebracht werden, um den zweiten Manschetten-Öffnungsbereich dauerhaft zu verschließen. Das Verschlussmittel umfasst vorzugsweise ein Haftmittel, ein Klebemittel, einen Klebestreifen, einen Zip-Verschluss oder einen Druckverschluss. Wird das flexible Zwischenelement, insbesondere zusammen mit dem Einfülltrichter, abgelöst, so kann der Automatikverschluss das Verschlussmittel mit sich selbst in Kontakt bringen bzw. einen auf der Innenseite der flexiblen Manschette angeordneten Klebestreifen mit sich selbst verkleben. Vorzugsweise haftet das Verschlussmittel an sich selbst dauerhaft. Dieser Mechanismus wird im Folgenden auch als Sicherheitsverschluss bezeichnet. Der zweite Manschetten-Öffnungsbereich kann einerseits mittels des Automatikverschlusses leicht öffenbar verschlossen werden und andererseits mit dem Sicherheitsverschluss einmalig und dauerhaft verschlossen werden. Der Sicherheitsverschluss ist insbesondere nach abschließendem Gebrauch der Auffangvorrichtung von Vorteil, um die aufgefangenen Ausscheidungen sicher bzw. fluiddicht bis zu einer Entsorgung im Auffangbeutel zu halten.

Gemäß einer weiteren bevorzugten Ausführungsform kann der Einfülltrichter einen zweiten Einfülltrichter-Öffnungsbereichs umfassen, der den Einfülltrichter antiparallel zum ersten Einfülltrichter-Öffnungsbereich oder liegend-S-förmig schneidet. Der zweite Einfülltrichter-Öffnungsbereich ist durch den antiparallelen Verlauf vorteilhaft größer als der erste Einfülltrichter-Öffnungsbereich bzw. umgibt eine größere Öffnung als der erste Einfülltrichter-Öffnungsbereich, bildet also einen Trichter. Dadurch wird ein Verschütten beim Auffangen der Ausscheidungen verringert oder verhindert. Durch den antiparallelen Verlauf kann zugleich der Umfang des Einfülltrichters entlang der Längsachse konstant bleiben, wodurch radiale Abmessungen des Einfülltrichters, der flexiblen Manschette und des Auffangbeutels fluchten. Im flachgedrückten Zustand kann die Auffangvorrichtung mangels etwaiger vorstehender Teile und damit anders als bei bekannten Vorrichtung sehr kompakt und platzsparend ausgelegt und insbesondere zusammengefaltet werden. Der liegend-S-förmige Verlauf des zweiten Einfülltrichter-Öffnungsbereichs, d.h. ein Verlauf wie ein liegender Buchstabe S, erzielt ebenfalls eine größere Öffnung als der erste Einfülltrichter-Öffnungsbereich, bildet also einen Trichter. Zusätzlich erlaubt der liegend-S-förmige Verlauf eine bessere Anpassung an die weibliche Anatomie, um das Verschütten bei der Aufnahme von Urin zu verringern.

Gemäß einer weiteren bevorzugten Ausführungsform kann der Auffangbeutel einen zweiten Auffangbeutel-Öffnungsbereich und einen Klebestreifen umfassen, wobei der Klebestreifen den zweiten Auffangbeutel-Öffnungsbereich öffenbar verschließt. Im Folgenden wird dieser Mechanismus auch als Entleerungsschlitz oder als Entsorgungsmechanismus bezeichnet. Ist das Auffangen von Ausscheidungen abgeschlossen und der Auffangbeutel durch den Sicherheitsverschluss einmalig und dauerhaft verschlossen, so ist es von Vorteil, die Ausscheidungen fluiddicht zu einem Abfluss oder zu einer Toilette transportieren zu können und dort den zweiten Auffangbeutel-Öffnungsbereich durch ein Abziehen des Klebestreifens zu öffnen und die Ausscheidungen kontrolliert aus dem Auffangbeutel zu entleeren. Anders als in den meisten bekannten Vorrichtungen ist durch den Entsorgungsmechanismus kein Geliermittel erforderlich, denn die Ausscheidungen können getrennt von der entleerten Auffangvorrichtung und ordnungsgemäß entsorgt werden. Dies ist umweltfreundlicher als bei bekannten Vorrichtungen, die inklusive gelierter Ausscheidungen entsorgt werden müssen.

Gemäß einer weiteren bevorzugten Ausführungsform kann die Federspange im gestauchten Zustand die flexible Manschette und den Einfülltrichter zylindrisch formen und im entspannten Zustand die flexible Manschette und den Einfülltrichter flach formen. So kann die erfindungsgemäße Auffangvorrichtung vorteilhaft im entspannten Zustand flach verstaut und/oder zusammengefaltet werden. Im gestauchten Zustand kann die Federspange die flexible Manschette und/oder den Einfülltrichter und/oder den Auffangbeutel zylindrisch formen, wodurch die erfindungsgemäße Auffangvorrichtung vorteilhaft verschlossen bzw. geöffnet werden kann. Ein Benutzer kann durch Drücken der Federspange an ihren distalen Enden bzw. diametral, etwa zwischen Daumen und Zeigefinger derselben Hand, den gestauchten Zustand und durch Loslassen den entspannten Zustand herbeiführen. So können die flexible Manschette bzw. die erfindungsgemäße Auffangvorrichtung einhändig schnell, sicher und einfach geöffnet und wieder verschlossen werden, um zum Beispiel ein zwischenzeitliches Auslaufen von aufgefangenen Ausscheidungen zu verhindern und/oder unangenehme Gerüche zu verringern.

Gemäß einer weiteren bevorzugten Ausführungsform kann die Auffangvorrichtung eine Zickzackfalzung aufweisen. Die Zickzackfalzung umfasst insbesondere mehrere senkrecht zur Längsachse ausgerichtete Falze, die entlang der Auffangvorrichtung in Abständen zueinander angeordnet sind. Dadurch kann die Auffangvorrichtung einerseits kompakt, z.B. in Taschenformat, bereitgestellt werden und andererseits bei Bedarf schnell und einfach, insbesondere nur mit einer Hand, entfaltet werden. Gerade bei dringendem Harndrang oder Brechreiz ist dies vorteilhaft. Des Weiteren ist es bevorzugt, dass das flexible Zwischenelement einen Falz bildet. Das flexible Zwischenelement kann als Gelenk zwischen Einfülltrichter und flexibler Manschette wirken. Dadurch können Falze am Einfülltrichter und an der flexiblen Manschette vermieden werden, sodass der Einfülltrichter und die flexible Manschette stabil bleiben. Das flexible Zwischenelement ermöglicht vorteilhaft die Auffangvorrichtung kompakt zusammenfalten zu können. Vorzugsweise bildet das flexible Zwischenelement einen Wickelfalz. Die erfindungsgemäße Auffangvorrichtung kann eine Kombination aus Zickzackfalzung und Wickelfalz aufweisen.

Gemäß einer weiteren bevorzugten Ausführungsform kann der Auffangbeutel ein intransparentes, fluiddichtes Kunststoffmaterial umfassen. Die Intransparenz ermöglicht es aufgefangene Ausscheidungen dezent und unauffällig zu einem Abfluss oder zu einer Toilette zu transportieren. Wenn das Kunststoffmaterial fluiddicht, d.h. insbesondere flüssigkeits- und geruchsdicht ausgelegt ist, so ist, anders als im Stand der Technik, vorteilhaft kein auslaufhinderndes Geliermittel zum Gelieren der Ausscheidungen erforderlich.

### Kurzbeschreibung der Zeichnungen

Ausführungsbeispiele der Erfindung werden anhand der Zeichnungen und der nachfolgenden Beschreibung näher erläutert. Es zeigen:
- Figur 1: ein Ausführungsbeispiel einer Auffangvorrichtung gemäß der Erfindung,
- Figur 2: ein weiteres Ausführungsbeispiel der Auffangvorrichtung in einem flachgedrückten Zustand gemäß der Erfindung,
- Figur 3A: ein weiteres Ausführungsbeispiel der Auffangvorrichtung mit Zickzackfalzung gemäß der Erfindung und
- Figur 3B: ein weiteres Ausführungsbeispiel der Auffangvorrichtung mit kombinierter Zickzack- und Wickelfalzung gemäß der Erfindung.

### Ausführungsformen der Erfindung

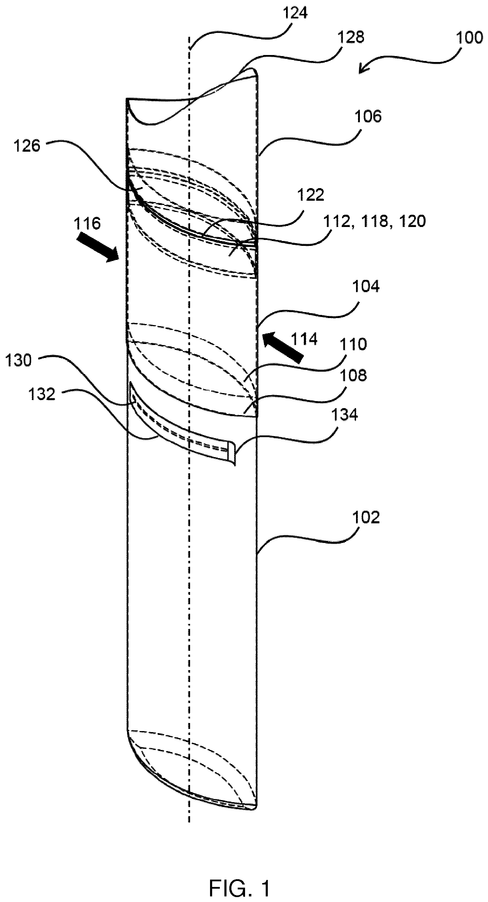

In der Figur 1 ist eine Auffangvorrichtung 100 zum Auffangen von Ausscheidungen, z.B. Urin oder Erbrochenes, in einem auseinandergefalteten und gestauchten Zustand gezeigt. Die Auffangvorrichtung 100 umfasst einen fluiddichten Auffangbeutel 102, eine flexible Karton-Manschette 104 und einen Einfülltrichter 106. Der Auffangbeutel 102 ist in dem auseinander gefalteten und gestauchten Zustand oval-zylinderförmig und hat eine ersten Auffangbeutel-Öffnungsbereich 108. Der ersten Auffangbeutel-Öffnungsbereich 108 überlappt an einer Innenseite eines ersten Manschetten-Öffnungsbereichs 110. Die Öffnungsbereiche 108 und 110 sind fluiddicht miteinander verklebt. An einer Innenseite der Manschette 104 ist eine Federspange 112 eingeklebt. Die Federspange 112 umfasst ein Federelement mit zwei Schenkeln, die in einem entspannten Zustand im Wesentlichen parallel zueinander angeordnet sind und in einem gestauchten Zustand eine O-Form bzw. eine Ellipsen-Form bilden. Im entspannten Zustand formt die Federspange 112 die Manschette 104, den Einfülltrichter 106 und den Auffangbeutel 102 flach. Im gestauchten Zustand formt die Federspange 112 die Manschette 104, den Einfülltrichter 106 und den Auffangbeutel 102 oval-zylinderförmig, wodurch die Auffangvorrichtung 100 verschlossen bzw. geöffnet wird. Ein Benutzer kann durch Drücken der Federspange 112 an ihren distalen Enden - durch Pfeile 114, 116 gezeigt - etwa zwischen Daumen und Zeigefinger derselben Hand, den gestauchten Zustand und durch Loslassen den entspannten Zustand herbeiführen. So ist die Manschette 104 bzw. die Auffangvorrichtung 100 einhändig sicher und einfach öffenbar und wieder verschließbar, um ein zwischenzeitliches Auslaufen von aufgefangenen Ausscheidungen zu verhindern und unangenehme Gerüche zu verringern. Dieser Mechanismus wird im Folgenden auch als Automatikverschluss bezeichnet. Die Manschette 104 dient als Griff zum Festhalten der Auffangvorrichtung 100 durch einen Benutzer, zu einem Einleiten bzw. Führen von Ausscheidungen in den Auffangbeutel 102 und zu einem Stabilisieren des ersten Auffangbeutel-Öffnungsbereichs 108. Die Manschette 104 umfasst außerdem einen zweiten Manschetten-Öffnungsbereich 118, an dessen Innenseite ist ein Klebeverschluss 120 angeordnet. An dem Klebeverschluss 120 ist eine flexibles Kunststoffband 122 ablösbar angeklebt. Das flexible Kunststoffband 122 steht aus dem zweiten Manschetten-Öffnungsbereich 118 entlang einer Längsachse 124 vor und überlappt an einer Innenseite eines ersten Einfülltrichter-Öffnungsbereichs 126 des Einfülltrichters 106. Das flexible Kunststoffband 122 ist am ersten Einfülltrichter-Öffnungsbereich 126 festgeklebt. Der Einfülltrichter 106 umfasst ein nanobeschichtetes Wachspapiermaterial, um wasser- bzw. schmutzabweisend zu sein. Der Auffangbeutel 102, die Manschette 104 und der Einfülltrichter 106 sind vorzugsweise entlang der Längsachse 124 angeordnet.

Wird das flexible Kunststoffband 122 samt Einfülltrichter 106 vom Klebeverschluss 120 abgelöst, bringt der Automatikverschluss im entspannten Zustand den Klebeverschluss 120 mit sich selbst in Kontakt und verschließt dadurch den zweiten Manschetten-Öffnungsbereich 118. Der Klebeverschluss 120 haftet an sich selbst dauerhaft. Dieser Mechanismus wird im Folgenden auch als Sicherheitsverschluss bezeichnet. Der zweite Manschetten-Öffnungsbereich 118 kann einerseits mittels des Automatikverschlusses leicht öffenbar verschlossen werden und andererseits mit dem Sicherheitsverschluss einmalig und dauerhaft verschlossen werden. Der Sicherheitsverschluss bewirkt nach abschließendem Gebrauch der Auffangvorrichtung 100, dass die aufgefangenen Ausscheidungen sicher bzw. fluiddicht bis zu einer Entsorgung im Auffangbeutel 102 gehalten werden.

Der Einfülltrichter 106 umfasst einen zweiten Einfülltrichter-Öffnungsbereich 128, der den Einfülltrichter 106 liegend-S-förmig schneidet. Der zweite Einfülltrichter-Öffnungsbereich 128 ist dadurch größer als der erste Einfülltrichter-Öffnungsbereich 126 bzw. umgibt eine größere Öffnung als der erste Einfülltrichter-Öffnungsbereich 126, bildet also einen Trichter. Dadurch wird ein Verschütten beim Auffangen der Ausscheidungen verringert oder verhindert. Durch den liegend-S-förmigen Verlauf kann zugleich der Umfang des Einfülltrichters 106 entlang der Längsachse 124 konstant bleiben, wodurch radiale Abmessungen des Einfülltrichters 106, der Manschette 104 und des Auffangbeutels 102 fluchten. Im entspannten Zustand kann die Auffangvorrichtung 100 mangels etwaiger vorstehender Teile, und damit anders als bei bekannten Vorrichtung, sehr flach, kompakt und platzsparend ausgelegt und insbesondere zusammengefaltet werden; der Zustand wir im Folgenden auch als flachgedrückter Zustand bezeichnet. Der liegend-S-förmige Verlauf erlaubt eine bessere Anpassung an die weibliche Anatomie, um das Verschütten bei der Aufnahme von Urin zu verringern.

Der Auffangbeutel 102 weist einen zweiten Auffangbeutel-Öffnungsbereich 130 und einen Klebestreifen 132 auf, wobei der Klebestreifen 132 den zweiten Auffangbeutel-Öffnungsbereich 130 öffenbar verschließt. Im Folgenden wird dieser Mechanismus auch als Entleerungsschlitz oder als Entsorgungsmechanismus bezeichnet. Ist das Auffangen von Ausscheidungen abgeschlossen und der Auffangbeutel 102 durch den Sicherheitsverschluss einmalig und dauerhaft verschlossen, so können die Ausscheidungen fluiddicht zu einem Abfluss oder zu einer Toilette transportiert und dort der zweite Auffangbeutel-Öffnungsbereich 130 durch ein Abziehen des Klebestreifens 132 geöffnet und die Ausscheidungen kontrolliert aus dem Auffangbeutel 102 entleert werden. Der Klebestreifen 132 weist einen abstehenden Endabschnitt 134 auf, an dem der Klebestreifen 132 zum Abziehen festgehalten werden kann.

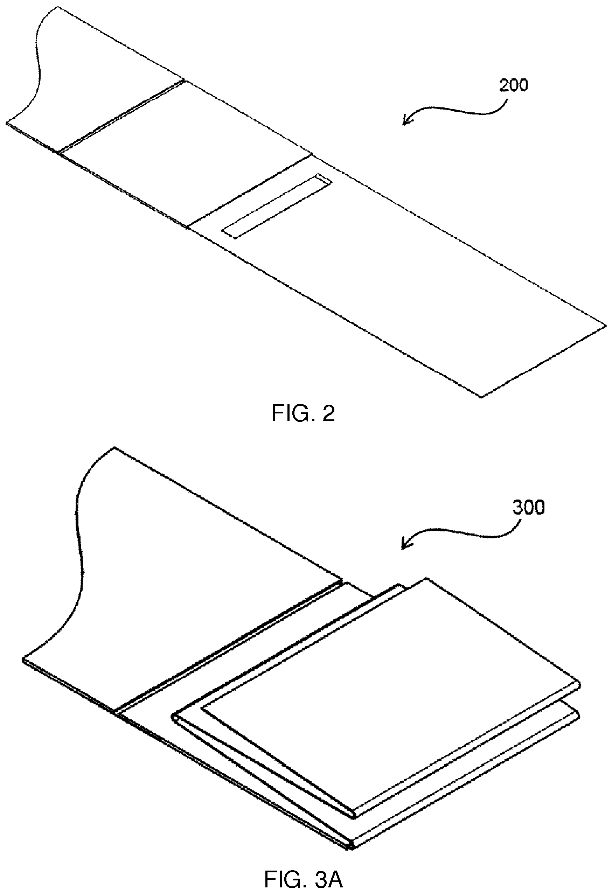

In der Figur 2 ist eine Auffangvorrichtung 200 in einem auseinandergefalteten und flachgedrückten bzw. entspannten Zustand gezeigt.

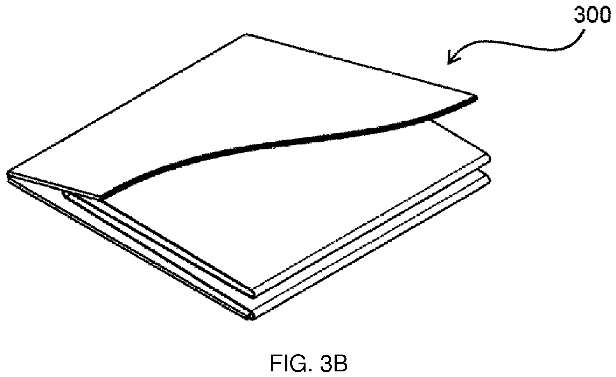

In der Figur 3A ist eine Auffangvorrichtung 300 mit einer Zickzackfalzung gezeigt. In der Figur 3B ist die Auffangvorrichtung 300 mit einer kombinierten Zickzack- und Wickelfalzung gezeigt. Die Zickzackfalzung umfasst mehrere Falze, die entlang der Auffangvorrichtung 300 in Abständen zueinander angeordnet sind. Dadurch kann die Auffangvorrichtung 300 einerseits kompakt, z.B. in Taschenformat, bereitgestellt werden und andererseits bei Bedarf schnell und einfach, insbesondere nur mit einer Hand, entfaltet werden. Gerade bei dringendem Harndrang oder Brechreiz ist dies von Vorteil.

### Bezugszeichenliste

- 100: Auffangvorrichtung
- 102: Auffangbeutel
- 104: Manschette
- 106: Einfülltrichter
- 108: Erster Auffangbeutel-Öffnungsbereich
- 110: Erster Manschetten-Öffnungsbereich
- 112: Federspange
- 114: Pfeil
- 116: Pfeil
- 118: Zweiter Manschetten-Öffnungsbereich
- 120: Klebeverschluss
- 122: Flexibles Kunststoffband
- 124: Längsachse
- 126: Erster Einfülltrichter-Öffnungsbereich
- 128: Zweiter Einfülltrichter-Öffnungsbereich
- 130: Zweiter Auffangbeutel-Öffnungsbereich
- 132: Klebestreifen
- 134: Endabschnitt
- 200: Auffangvorrichtung
- 300: Auffangvorrichtung

## Patentansprüche

1. Auffangvorrichtung (100; 200; 300) für Ausscheidungen mit:
einem Auffangbeutel (102) mit einem ersten Auffangbeutel-Öffnungsbereich (108);
einer flexiblen Manschette (104) mit einem ersten (110) und einem zweiten Manschetten-Öffnungsbereich (118),
wobei der erste Auffangbeutel-Öffnungsbereich (108) dem ersten Manschetten-Öffnungsbereich (110) gegenüberliegend angeordnet ist, und
wobei die flexible Manschette (104) eine Federspange (112) umfasst, die die flexible Manschette (104) öffenbar verschließt, wobei die Federspange zwei Schenkel aufweist, die in einem entspannten Zustand parallel zueinander angeordnet sind und in einem gestauchten Zustand eine O-Form bilden, und
einem Einfülltrichter (106), der einen ersten Einfülltrichter-Öffnungsbereich (126) aufweist, wobei der erste Einfülltrichter-Öffnungsbereich (126) dem zweiten Manschetten-Öffnungsbereich (118) gegenüberliegend angeordnet ist
**dadurch gekennzeichnet, dass**
die Auffangvorrichtung (100; 200; 300) ein flexibles Zwischenelement (122) umfasst, das den Einfülltrichter (106) mit dem zweiten Manschetten-Öffnungsbereich (118) verbindet, wobei das flexible Zwischenelement (122) mit dem zweiten Manschetten-Öffnungsbereich (118) ablösbar verbunden ist und wobei das flexible Zwischenelement ein fluiddichtes, biegsames Kunststoffband umfasst, das an einer Innenseite der flexiblen Manschette und an einer Innenseite des Einfülltrichters angeordnet ist.

2. Auffangvorrichtung (100; 200; 300) für Ausscheidungen nach Anspruch 1
**dadurch gekennzeichnet, dass**
die flexible Manschette (104) ein Verschlussmittel (120) umfasst, das das flexible Zwischenelement (122) in einem abgelösten Zustand freilegt, um den zweiten Manschetten-Öffnungsbereich (118) dauerhaft zu verschließen.

3. Auffangvorrichtung (100; 200; 300) für Ausscheidungen nach einem der vorhergehenden Ansprüche
**dadurch gekennzeichnet, dass**
der Einfülltrichter (106) einen zweiten Einfülltrichter-Öffnungsbereichs (128) umfasst, der den Einfülltrichter (106) antiparallel zum ersten Einfülltrichter-Öffnungsbereich (126) oder liegend-S-förmig schneidet.

4. Auffangvorrichtung (100; 200; 300) für Ausscheidungen nach einem der vorhergehenden Ansprüche
**dadurch gekennzeichnet, dass**
der Auffangbeutel (102) einen zweiten Auffangbeutel-Öffnungsbereich (130) und einen Klebestreifen (132) umfasst, wobei der Klebestreifen (132) den zweiten Auffangbeutel-Öffnungsbereich (130) öffenbar verschließt.

5. Auffangvorrichtung (100; 200; 300) für Ausscheidungen nach einem der vorhergehenden Ansprüche
**dadurch gekennzeichnet, dass**
die Federspange (112) in einem gestauchten Zustand die flexible Manschette (104) und den Einfülltrichter (106) zylinderartig formt und in einem entspannten Zustand die flexible Manschette (104) und den Einfülltrichter (106) flach formt.

6. Auffangvorrichtung (100; 200; 300) für Ausscheidungen nach einem der vorhergehenden Ansprüche
**dadurch gekennzeichnet, dass**
die Auffangvorrichtung (100; 200; 300) eine Zickzackfalzung aufweist.

7. Auffangvorrichtung (100; 200; 300) für Ausscheidungen nach einem der vorhergehenden Ansprüche
**dadurch gekennzeichnet, dass**
das flexible Zwischenelement (122) einen Falz bildet.

8. Auffangvorrichtung (100; 200; 300) für Ausscheidungen nach einem der vorhergehenden Ansprüche
**dadurch gekennzeichnet, dass**
der Auffangbeutel (102) ein intransparentes, fluiddichtes Kunststoffmaterial umfasst.

## Claims

1. A collection device (100; 200; 300) for excretions, comprising:
a collection bag (102) having a first collection bag opening region (108);
a flexible collar (104) having a first (110) and a second collar opening region (118),
the first collection bag opening region (108) being arranged opposite the first collar opening region (110), and
the flexible collar (104) comprising a spring clasp (112) that openably closes the flexible collar (104), the spring clasp having two legs which, in a relaxed state, are arranged parallel to one another and, in a compressed state, form an O-shape, and
a feed funnel (106) which has a first feed funnel opening region (126), the first feed funnel opening region (126) being arranged opposite the second collar opening region (118),
**characterized in that**
the collection device (100; 200; 300) comprises a flexible intermediate element (122) that connects the feed funnel (106) to the second collar opening region (118), the flexible intermediate element (122) being detachably connected to the second collar opening region (118), and the flexible intermediate element comprising a fluid-tight, bendable plastics strip which is arranged on an inner side of the flexible collar and on an inner side of the feed funnel.

2. The collection device (100; 200; 300) for excretions according to claim 1,
**characterized in that**
the flexible collar (104) comprises a closure means (120) that the flexible intermediate element (122) exposes when in a detached state, in order to permanently close off the second collar opening region (118).

3. The collection device (100; 200; 300) for excretions according to either of the preceding claims,
**characterized in that**
the feed funnel (106) comprises a second feed funnel opening region (128) that intersects the feed funnel (106) anti-parallel to the first feed funnel opening region (126) or in a lying S-shape.

4. The collection device (100; 200; 300) for excretions according to any of the preceding claims,
**characterized in that**
the collection bag (102) comprises a second collection bag opening region (130) and an adhesive strip (132), the adhesive strip (132) openably closing the second collection bag opening region (130).

5. The collection device (100; 200; 300) for excretions according to any of the preceding claims,
**characterized in that**
the spring clasp (112), in a compressed state, makes the flexible collar (104) and the feed funnel (106) a cylindrical shape and, in a relaxed state, makes the flexible collar (104) and the feed funnel (106) flat.

6. The collection device (100; 200; 300) for excretions according to any of the preceding claims,
**characterized in that**
the collection device (100; 200; 300) is folded in a zig-zag shape.

7. The collection device (100; 200; 300) for excretions according to any of the preceding claims,
**characterized in that**
the flexible intermediate element (122) forms a fold.

8. The collection device (100; 200; 300) for excretions according to any of the preceding claims,
**characterized in that**
the collection bag (102) comprises a non-transparent, fluid-tight plastics material.

## Revendications

1. Dispositif collecteur (100 ; 200 ; 300) pour excréments comprenant :
un sac collecteur (102) avec une première zone d'ouverture de sac collecteur (108) ;
une manchette flexible (104) avec un première (110) et une seconde zone d'ouverture de manchette (118),
dans lequel la première zone d'ouverture de sac collecteur (108) est agencée en regard de la première zone d'ouverture de manchette (110) et
dans lequel la manchette flexible (104) comprend une agrafe élastique (112), qui ferme la manchette flexible (104) de manière à pouvoir l'ouvrir, dans lequel l'agrafe élastique présente deux branches, qui sont agencées parallèlement l'une à l'autre dans un état détendu et forment un O dans un état comprimé et
une trémie de remplissage (106), qui présente une première zone d'ouverture de trémie de remplissage (126), dans lequel la première zone d'ouverture de trémie de remplissage (126) est agencée en regard de la seconde zone d'ouverture de manchette (118)
**caractérisé en ce que**
le dispositif collecteur (100 ; 200 ; 300) comprend un élément intermédiaire flexible (122), qui relie la trémie de remplissage (106) avec la seconde zone d'ouverture de manchette (118), dans lequel l'élément intermédiaire flexible (122) est relié de manière détachable avec la seconde zone d'ouverture de manchette (118) et dans lequel l'élément intermédiaire flexible présente une bande en matières plastiques étanche aux fluides et souple, qui est agencée sur un côté interne de la manchette flexible et sur un côté interne de la trémie de remplissage.

2. Dispositif collecteur (100 ; 200 ; 300) pour excréments selon la revendication 1
**caractérisé en ce que**
la manchette flexible (104) comprend un moyen de fermeture (120), qui expose l'élément intermédiaire (122) dans un état désolidarisé, afin de fermer de manière durable la seconde zone d'ouverture de manchette (118).

3. Dispositif collecteur (100 ; 200 ; 300) pour excréments selon l'une quelconque des revendications précédentes
**caractérisé en ce que**
la trémie de remplissage (106) comprend une seconde zone d'ouverture de trémie de remplissage (128), qui coupe la trémie de remplissage (106) de manière antiparallèle par rapport à la première zone d'ouverture de trémie de remplissage (126) ou sous la forme d'un S couché.

4. Dispositif collecteur (100 ; 200 ; 300) pour,excréments selon l'une quelconque des revendications précédentes
**caractérisé en ce que**
le sac collecteur (102) comprend une seconde zone d'ouverture de sac collecteur (130) et un ruban adhésif (132), dans lequel le ruban adhésif (132) ferme la seconde zone d'ouverture de sac collecteur (130) de manière à pouvoir l'ouvrir.

5. Dispositif collecteur (100 ; 200 ; 300) pour excréments selon l'une quelconque des revendications précédentes
**caractérisé en ce que**
l'agrafe élastique (112) déforme la manchette flexible (104) et la trémie de remplissage (106) en forme de cylindre dans un état comprimé et déforme à plat la manchette flexible (104) et la trémie de remplissage (106) dans un état détendu.

6. Dispositif collecteur (100 ; 200 ; 300) pour excréments selon l'une quelconque des revendications précédentes
**caractérisé en ce que**
le dispositif collecteur (100 ; 200 ; 300) présente un pliage en accordéon.

7. Dispositif collecteur (100 ; 200 ; 300) pour excréments selon l'une quelconque des revendications précédentes
**caractérisé en ce que**
l'élément intermédiaire flexible (122) forme un pli.

8. Dispositif collecteur (100 ; 200 ; 300) pour excréments selon l'une quelconque des revendications précédentes
**caractérisé en ce que**
le sac collecteur (102) comprend un matériau plastique étanche aux fluides et opaque.
